(19) Europäisches Patentamt
European Patent Office
Office européen des brevets

(11) **EP 1 219 234 A1**

(12) **EUROPEAN PATENT APPLICATION**

(43) Date of publication:
03.07.2002 Bulletin 2002/27

(51) Int Cl.7: **A61B 5/00**

(21) Application number: 01310531.7

(22) Date of filing: 17.12.2001

(84) Designated Contracting States:
**AT BE CH CY DE DK ES FI FR GB GR IE IT LI LU MC NL PT SE TR**
Designated Extension States:
**AL LT LV MK RO SI**

(30) Priority: 29.12.2000 US 751681

(71) Applicant: **GE Medical Systems Information Technologies, Inc.**
Milwaukee, Wisconsin 53223 (US)

(72) Inventors:
• **Lovejoy, David Anthony**
Thiensville, Wisconsin 53092 (US)
• **Byers, George Alexander**
Milwaukee, Wisconsin 53223-4375 (US)

(74) Representative: **Goode, Ian Roy**
**GE LONDON PATENT OPERATION,**
**Essex House,**
**12/13 Essex Street**
**London WC2R 3AA (GB)**

(54) **Method and apparatus for assessing tissue perfusion**

(57) A method and apparatus for assessing tissue perfusion of a patient. The apparatus includes a probe (12) for contacting the mucosa tissue in the upper respiratory/digestive tract of the patient (10), and a sensor (30) coupled to the probe (12) for directly detecting a pH measurement of the mucosa tissue and for generating an electrical signal in response to the detected pH measurement.

For the method of the invention, a probe (12) capable of measuring pH is provided. The probe (12) is placed in contact with the mucosa tissue in the upper respiratory/digestive tract of the patient. A pH measurement of the mucosa tissue is obtained. The pH measurement is converted into an indicator to a clinician representing a level of tissue perfusion.

Fig. 1

EP 1 219 234 A1

**Description**

The invention relates to assessing perfusion failure of a patient by measuring the pH of the mucosa tissue in the upper digestive/respiratory tract of the patient.

Perfusion is defined as the flow of blood through the body to organs or tissues to supply nutrients and oxygen. Perfusion failure occurs when the flow of blood through the body is disrupted. Specifically, perfusion failure may be caused by bacteria and infection, by hemorrhage, or by coronary syndromes, such as myocardial infarction. Perfusion failure leads to the progressive deterioration of the cardiovascular functions of the body. If the perfusion failure can be corrected by the appropriate therapy, the perfusion failure is referred to as refractory shock. However, if the perfusion failure is irreversible and ultimately lethal, the perfusion failure is referred to as irreversible shock. Methods have been developed to detect and correct perfusion failure with the appropriate therapy during refractory shock, before the patient's condition deteriorates to irreversible shock.

Perfusion failure can be detected by measuring systemic levels of blood gases. Systemic levels of blood gases provide an indication of perfusion failure throughout the body. One method of determining perfusion failure by measuring systemic levels of blood gases is to perform an arterial blood gas (ABG) analysis of the patient's blood. The ABG analysis produces five main measurements, including arterial pH, the arterial partial pressure of oxygen ($P_{aO2}$), the arterial partial pressure of carbon dioxide ($P_{aCO2}$), oxygen saturation ($S_{O2}$), and bicarbonate ($HCO_3^-$) concentration. Although the ABG analysis is considered the most accurate way of detecting perfusion failure, it has several drawbacks. First, since the patient's blood must be drawn, the ABG analysis is highly invasive. Second, the ABG analysis only gives information intermittently when blood is drawn from the patient and then analyzed. Third, there is a substantial delay between the time the patient's blood is drawn and the time the ABG analysis results are available.

Another method of determining perfusion failure by measuring systemic levels of blood gases is the use of a pulmonary artery catheter. For this method, a catheter is inserted directly into the pulmonary artery to take hemodynamic measurements, such as cardiac output, pulmonary artery occlusion pressure, and mixed venous oxygen saturation, along with blood gas measurements, such as arterial oxygenation, global oxygen delivery, and oxygen consumption. Even though these measurements provide useful information about systemic perfusion failure, the patient's condition may deteriorate considerably before changes in the systemic levels of blood gases reflect inadequate perfusion. Moreover, pulmonary artery catheters are highly invasive and have been associated with increased mortality rates, due to unintended arrhythmias, hemorrhage, thromboembolism, sepsis, and endocardial damage.

Perfusion failure can also be detected by measuring the by-products of anaerobic metabolism in bodily tissues. The by-products of anaerobic metabolism are not carried away from bodily tissues as quickly during low blood flow states as during normal blood flow states. The build-up of by-products results in tissue acidosis, which is reflected in a decrease in the pH level of the tissue. Accordingly, a decrease in tissue pH correlates to perfusion failure.

One method of determining perfusion failure by measuring the by-products of anaerobic metabolism is gastric tonometry. Gastric tonometry takes advantage of the fact that during perfusion failure blood flow is directed away from the gastrointestinal (GI) tract to organs such as the heart and the brain. Gastric tonometry involves inserting a catheter down a nasal gastric tube into the patient's stomach. The catheter contains a gas-permeable silicone balloon filled with saline. While in the patient's stomach, the saline in the balloon is allowed to equilibrate with carbon dioxide, a by-product of anaerobic metabolism. The catheter is removed from the patient's stomach, and the saline from the balloon is analyzed for its carbon dioxide level. Gastric tonometry provides accurate information about tissue-level perfusion failure, but it is a highly invasive procedure that requires a significant length of time for the saline in the balloon to equilibrate with the carbon dioxide in the stomach.

Another less-invasive method of detecting perfusion failure by measuring the by-products of anaerobic metabolism is disclosed in U.S. Patent No. 6,055,447. The '447 Patent discloses a method and apparatus for assessing impairment of blood circulation of a patient by measuring the partial pressure of carbon dioxide in the patient's upper digestive/respiratory tract.

During perfusion failure, the body automatically directs blood flow to the organs that need continuous blood flow to survive and away from the organs that do not need continuous blood flow to survive. As a result, the body directs blood flow to organs such as the heart, kidney, liver, adrenal glands, and brain, and away from the organs of the GI tract. With blood flow being directed away from the GI tract, the GI tract is the first portion of the body to suffer from inadequate tissue perfusion or ischemia. The term ischemia is defined as a decrease in the blood supply to a bodily organ, tissue, or part caused by constriction or obstruction of the blood vessels. Accordingly, if ischemia in the GI tract can be detected before systemic perfusion failure occurs, the clinician can take steps to prevent the patient from deteriorating into irreversible shock.

In general, ischemia can be detected in tissues by measuring the pH level of the tissue. During perfusion failure, lactic acid, a by-product of anaerobic metabolism, is not carried away from tissues as quickly as during normal perfusion. The lactic acid builds up in the tissue, increasing the acidosis of the tissue. This increase in the acidosis of the tissue is reflected in a decrease in the pH of the tissue. The pH of the tissue is determined directly based on the level of H+ ions in the tissue. The pH of the tissue is determined directly from $H^+$ ion concentration by the following equation:

$$pH = - \log [H^+]$$

The pH of the tissue can also be calculated indirectly from the partial pressure of carbon dioxide by the Henderson-Hasselbalch equation:

$$pH = 6.1 + (\log[HCO_3^-]/\alpha \, P_{CO2})$$

where 6.1 is the logarithm of a carbonic acid constant; [$HCO_3^-$] is the bicarbonate concentration of blood; $\alpha$ is a constant that relates the partial pressure of carbon dioxide to the concentration of carbon dioxide; and $P_{CO2}$ is the partial pressure of carbon dioxide.

In the GI tract, ischemia can be detected by determining intramucosal pH (pHi). The mucosa layer is the most proximal of the four layers of the GI tract. Below the mucosa layer is the submucosa layer. The arterial supply for GI tract perfusion flows through arterioles in the submucosa layer and through branches of the arterioles into the folds and projections, called villus, of the mucosa layer. Due to the flow of blood through the villus of the mucosa layer, ischemia of the GI tract can be detected by measuring the adequacy of perfusion in the tissue of the mucosa layer.

Intramucosal pH (pHi) can be determined indirectly by measuring the partial pressure of carbon dioxide in the mucosa tissue and then calculating the pH using the Henderson-Hasselbalch equation as follows:

$$pHi = 6.1 + (\log[HCO_3^-]/\alpha \, \text{mucosal } P_{CO2})$$

where pHi is the calculated intramucosal pH; 6.1 is the logarithm of a carbonic acid constant; [$HCO_3^-$] is the mucosal bicarbonate concentration, which is assumed to be equal to the arterial bicarbonate concentration; $\alpha$ is a constant that relates the mucosal partial pressure of carbon dioxide to the mucosal concentration of carbon dioxide; and mucosal $P_{CO2}$ is the partial pressure of carbon dioxide in the mucosa tissue.

Detecting ischemia by measuring the partial pressure of carbon dioxide and then calculating intramucosal pH with the Henderson-Hasselbalch equation is an indirect and possibly inaccurate method of detecting perfusion failure. The Henderson-Hasselbalch equation depends on two assumptions: first, that the intramucosal bicarbonate concentration is equal to the arterial bicarbonate concentration, and second, that the arterial bicarbonate concentration is constant. However, these assumptions are inaccurate during states of very low perfusion. Specifically, during partial or total GI tract ischemia, the mucosal bicarbonate concentration may be significantly lower than the arterial bicarbonate concentration. Moreover, the arterial bicarbonate concentration fluctuates significantly in very low perfusion states. As a result, the calculated intramucosal pH may be lower than the actual intramucosal pH. If the calculated intramucosal pH is lower than the actual intramucosal pH, perfusion failure may be indicated when perfusion failure is not actually occurring.

Thus, a need exists for a minimally invasive method of providing information about tissue-level perfusion failure based on a direct measurement of the tissue pH level, rather than on an indirect or highly invasive determination of tissue perfusion.

In order to avoid the inaccuracies associated with making a measurement of the partial pressure of carbon dioxide and then indirectly determining the pH level based on assumptions regarding the bicarbonate concentration, the present invention measures the $H^+$ ion concentration of the mucosa tissue directly. The pH is determined directly from the $H^+$ ion concentration for a more accurate result than when pH is calculated from the partial pressure of carbon dioxide.

Accordingly, the invention is embodied in a device for assessing perfusion failure of a patient, including a probe for contacting the mucosa tissue in the upper digestive/respiratory tract of a patient, and a sensor coupled to the probe for directly detecting the pH of the mucosa tissue. Preferably, the sensor is either an ion-selective, field-effect transistor or an electrochemical sensor. Preferably, the sensor is not encapsulated within a permeable membrane. The device includes a holder for the probe to secure the probe to the patient.

In another embodiment, the device includes a pH sensor and a second sensor for acquiring an end-tidal carbon-dioxide partial pressure measurement. The end-tidal carbon-dioxide partial-pressure measurement is used as a reference measurement to increase the accuracy of the pH measurement.

In still another embodiment, the device includes additional sensors or sensor platforms for detecting not only pH and end-tidal carbon-dioxide partial-pressure, but also for detecting blood chemistry data and saliva chemistry data.

The invention is also embodied in a new method of assessing tissue perfusion in a patient. The method includes the acts of providing a probe capable of measuring pH, placing the probe in contact with the mucosa tissue in the patient's upper digestive/respiratory tract, obtaining a pH measurement of the mucosa tissue, and converting the pH measurement into an indicator to a clinician representing the level of perfusion failure.

In another embodiment, the method includes the acts of providing a sensor capable of measuring end-tidal carbon-dioxide partial-pressure, placing the probe in the patient's upper digestive/respiratory tract, obtaining an end-tidal carbon-dioxide partial-pressure measurement, and comparing the pH measurement to the end-tidal carbon-dioxide partial-pressure measurement in order to increase the accuracy of the pH measurement.

In still another embodiment, the method includes the acts of providing additional sensors or sensor platforms for detecting not only pH and end-tidal carbon-dioxide partial-pressure, but also for detecting blood-chemistry data and saliva-chemistry data.

Embodiments of the invention will now be described, by way of example, with reference to the accompanying drawings, in which:

FIG. 1 illustrates a holder for a probe attached to a patient's mouth and coupled to a display unit.

FIG. 2 is a flow chart illustrating one preferred embodiment of the method of the invention.

FIG. 3 is a schematic illustrating the electrochemical embodiment of the sensor.

FIG. 4 is a schematic illustrating the ion-selective, field-effect transistor embodiment of the sensor.

FIG. 5 is a perspective view of the holder of FIG. 1.

FIG. 6 is a top view of the holder of FIG. 1.

FIG. 7 is a side view of the holder of FIG. 1.

FIG. 8 is a flow chart illustrating another preferred embodiment of the method of the invention.

Before one embodiment of the invention is explained in full detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including" and "comprising" and variations thereof herein is meant to encompass the items listed thereafter and equivalents thereof as well as additional items.

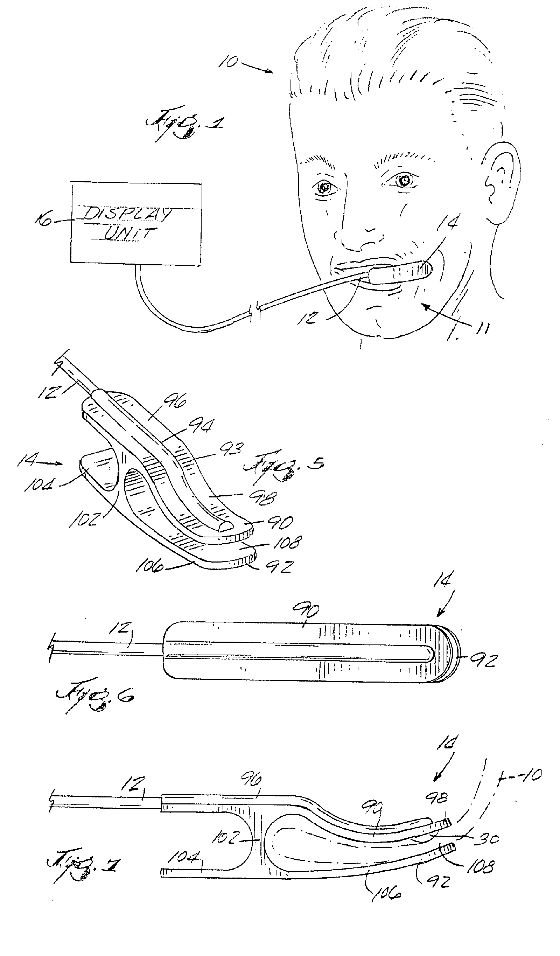

FIG. 1 illustrates a device 11 embodying the invention. The device 11 includes a probe 12, a sensor 30 (shown in FIG. 7) coupled to the probe 12, a holder 14 coupled to the probe 12, and a display unit 16 coupled to the probe 12. The display unit 16 may be a stand-alone unit dedicated solely to providing an indicator of tissue perfusion levels to a clinician. Alternatively, the display unit 16 may be part of a complete patient monitoring system.

Before the preferred embodiments of the sensor 30 are described in detail, it should be understood that the use of any sensor to detect the pH of the mucosa tissue in the upper digestive/respiratory tract in order to assess perfusion failure is considered within the scope of the invention.

In the most preferred embodiment of the invention, an ion-selective, field-effect transistor (ISFET) is used to detect the pH of the mucosa tissue. ISFETs are steady-state devices similar to metal-oxide semiconductor field-effect transistors (MOSFET). MOSFETs are composed of two diodes separated by a gate region. The gate is a thin insulator, usually silicon dioxide, upon which a metallic material is deposited. Voltage applied to the gate generates an electric field, and thus current flows between the drain and the source. ISFETs are similar to MOSFETS, except that the metal gate region is replaced with an ion-selective layer. Ions traveling through the ion-selective layer generate an electric field, and thus current flows between the drain and source.

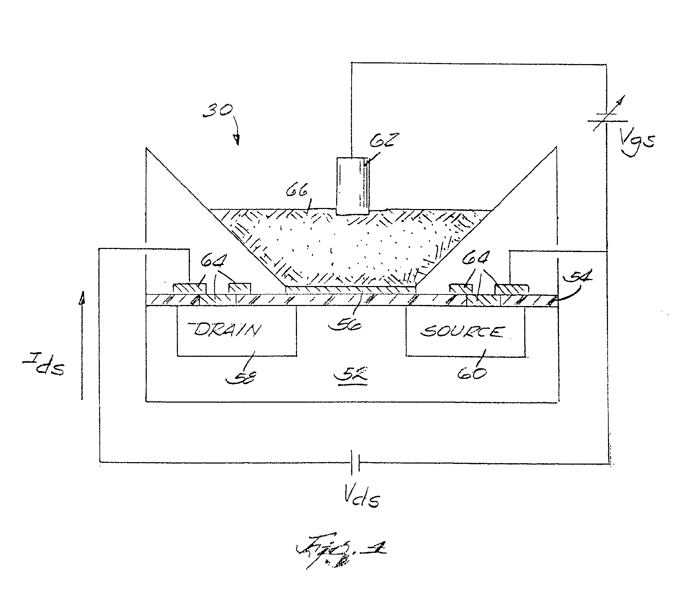

FIG. 4 is a schematic illustration of one embodiment of the sensor 30. The sensor 30 is an ISFET and includes a gate 52, an insulator 54, an ion-selective surface 56, a drain 58, a source 60, a reference electrode 62, and a plurality of metal contacts 64. The ion-selective surface 56 and the reference electrode 62 are in contact with the mucosa tissue 66. The reference electrode 62 keeps a constant voltage potential $V_{gs}$ against the mucosa tissue 66, independent of the mucosa tissue composition. As $H^+$ ions pass through the ion-selective surface 56, a voltage potential is developed at the ion-selective surface 56 in response to the $H^+$ ion concentration of the mucosa tissue 66. The ion-selective surface 56 is not encapsulated by any permeable membranes, rather the surface itself is ion-selective. The voltage potential $V_{ds}$ developed in response to the $H^+$ ion concentration modulates the current between the drain 58 and the source 60. As the voltage potential $V_{ds}$ across the gate changes, the ISFET current $I_{ds}$ flows. The voltage potential $V_{ds}$ correlates to the pH of the mucosa tissue.

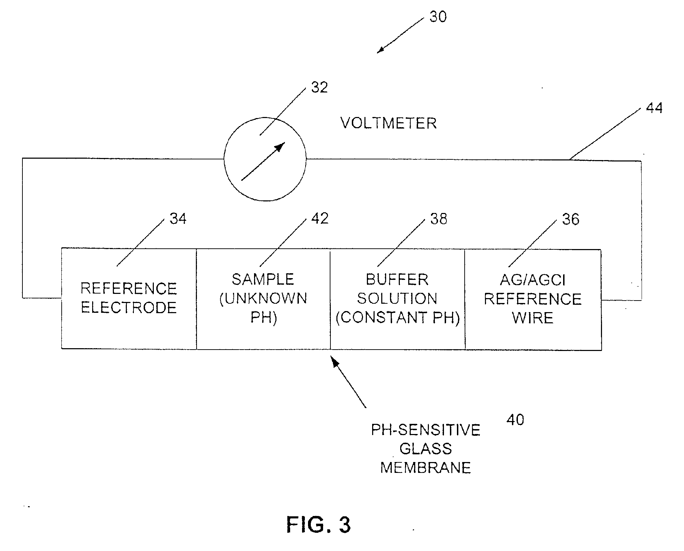

FIG. 3 is a schematic illustration of another suitable sensor 30. The sensor 30 shown in FIG. 3 is an electrochemical sensor. The electrochemical sensor 30 includes a voltmeter 32 coupled between a reference electrode 34 and a silver/silver-chloride reference wire 36. A shielded lead 44 connects the reference wire 36 to the voltmeter 32. The reference wire 36 is immersed within a buffer solution 38 with a constant pH level. Preferably, the buffer solution 38 is hydrochloric acid (HCl).

A pH-sensitive glass membrane 40 encapsulates the reference wire 36 and the buffer solution 38. Preferably, the glass membrane 40 is not encapsulated within an ion-selective permeable membrane. Ion-selective permeable membranes are not necessary in order to measure $H^+$ ion concentration, as they are necessary to measure carbon dioxide concentration and other ion concentrations. Rather than having an ion-selective permeable membrane, the glass membrane composition itself is ion-selective. Preferably, the ion-selective composition of the glass membrane 40 is $SiO_2$ or $Na_2O$.

The pH-sensitive glass membrane 40 and the reference electrode 34 are in contact with the mucosa tissue, represented by sample 42, which has an unknown pH. The reference electrode 34 keeps a constant voltage potential against the sample 42, independent of the sample composition. In response to the difference in pH of the buffer solution 38 and the sample 42, a direct voltage is developed between the inside and outside of the glass membrane 40. The voltage is caused by an ion exchange at each surface of the glass membrane 40 between the metal ions of the glass and the $H^+$ ions of the solutions. The approach of $H^+$ ions to the outside of the membrane 40 causes the silicate structure of the glass to conduct a positive charge into the buffer solution 38 inside the membrane 40. The ion exchange across the glass membrane 40 is controlled by the concentration of $H^+$ ions in each solution, and thus, the pH of each solution. The change in the voltage potential between the reference electrode 34 and the reference wire 36 is sensed by the voltmeter 32. If the concentration of $H^+$ ions in both solutions is the same, the potential difference across the glass membrane and the output of the voltmeter 32 is zero volts. Thus, the output of the voltmeter 32 correlates to the pH of the mucosa tissue, represented by sample 42.

In another preferred embodiment, the device 11 includes a sensor for acquiring an end-tidal carbon-dioxide partial-pressure ($P_{etCO2}$) measurement.

A $P_{etCO2}$ sensor suitable for use in the present invention is disclosed in U.S. Patent Application serial number 09/477,914 entitled "Low Cost Main Stream Gas Analyzer System".

In still another preferred embodiment of the invention, the device 11 includes additional sensors or sensor platforms for acquiring blood-chemistry and/or saliva-chemistry data from the patient. In order to implement the additional sensors into a platform sensor package, preferably one or more ISFETs capable of detecting multiple species of ions and molecules are fabricated onto one silicon chip.

Preferably, the additional sensors are capable of detecting the blood chemistry data that is normally gathered in an arterial blood gas (ABG) analysis. The additional sensors are preferably capable of detecting at least one of pH, the partial pressure of oxygen ($P_{O2}$), the partial pressure of carbon dioxide ($P_{CO2}$), bicarbonate, hematocrit, hemoglobin, oxygen saturation ($S_{O2}$), electrolyte concentration, and metabolite concentration. The additional sensors are preferably capable of detecting electrolytes including at least one of sodium, potassium, calcium, and chloride. The additional sensors are preferably capable of detecting metabolites including at least one of glucose, lactate, creatinine, and urea. The additional sensors are also preferably capable of detecting saliva chemistry data including at least one of cholesterol, lactate, electrolytes, illegal or abused drugs, glucose, bone markers, cystic fibrosis, HIV, and pregnancy.

FIGS. 5-7 illustrate the preferred embodiment of the holder 14. Before the preferred embodiment of the holder 14 is described in detail, it should be understood that the holder 14 could be constructed in any configuration and of any material capable of placing the sensor or sensors in contact with the mucosa tissue in the patient's upper digestive/respiratory tract.

Referring to FIGS. 5-7, the holder 14 preferably includes an inner holder portion 90 and an outer holder portion 92 coupled to the inner holder portion 90 by a resilient connecting member 102. The inner holder portion 90 includes an outer surface 93 and a groove 94 formed in the outer surface 93. The probe 12 is positioned within the groove 94. The inner holder portion 90 also includes a first end 96 and a second end 98. The first end 96 is a projection for use by the clinician to grasp the holder 14. The second end 98 is a gradually downward-sloping projection that is positioned within the patient's mouth, preferably in contact with the patient's cheek. As illustrated in FIG. 7, the sensor 30 is coupled to the second end 98. The sensor 30 is positioned to contact the mucosa tissue within the patient's mouth.

The outer holder portion 92 includes a first end 104 and a second end 106. The first end 104 is a projection for use by the clinician in conjunction with the first end 96 of the inner holder portion 92 to grasp the holder 14. The second end 106 is a gradually upward-sloping projection that is positioned outside the patient's mouth, preferably in contact with the cheek-area of the patient's face. The space 108 between the second end 98 of the inner holder portion 90 and the second end 106 of the outer holder portion 92 is such that the holder 14 remains secured to the patient's cheek.

Preferably, the holder 14 is constructed from a soft, pliable material that easily conforms to the patient's anatomy while remaining rigid enough to secure the holder 14 to the patient's face. Preferably, the material for the holder 14 is biocompatible.

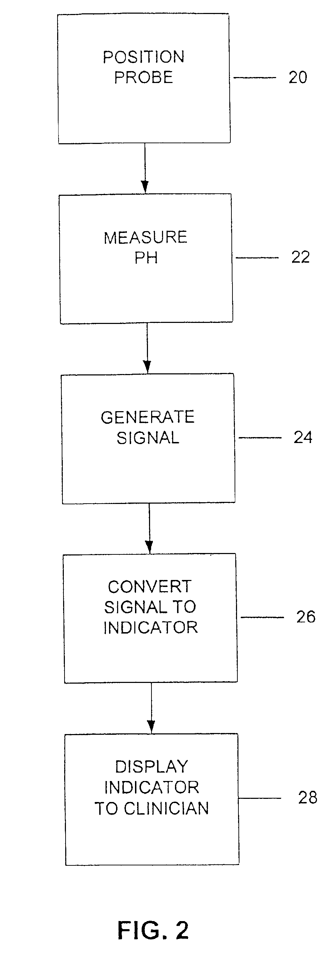

FIG. 2 is a flow chart illustrating the method of the invention. Referring to FIGS. 1 and 2, the holder 14 coupled to the probe 12 is positioned 20 on the patient. Preferably, the holder 14 is attached to the patient's cheek, and when in the appropriate position, presents the sensor 30 in contact with the oral mucosa tissue of the patient 10. In other embodiments (not shown), the holder 14 may be attached to the patient's lip or under the patient's tongue. Referring to FIGS. 5-7, in order to attach the holder 14, the clinician grasps the holder 14 by the first end 96 of the inner holder portion 90 and by the first end 104 of the outer holder portion 92. The clinician then positions the second end 98 of the inner holder portion 90 within the patient's mouth, preferably so that the sensor 30 is in contact with the mucosa tissue inside the patient's cheek. At the same time the clinician positions the second end 106 of the outer holder portion 92 outside the patient's mouth, preferably so that the second end 106 is in contact with the cheek area of the patient's face.

Still referring to FIGS. 1 and 2, the sensor 30 within the probe 12 measures 22 the pH by detecting the $H^+$ ion concentration of the mucosa tissue in the patient's mouth. The sensor 30 generates 24 an electrical signal in response to the detected pH. The signal is converted 26 into an indicator of the level of perfusion. The indicator of the level of perfusion is displayed 28 to a clinician on display unit 16.

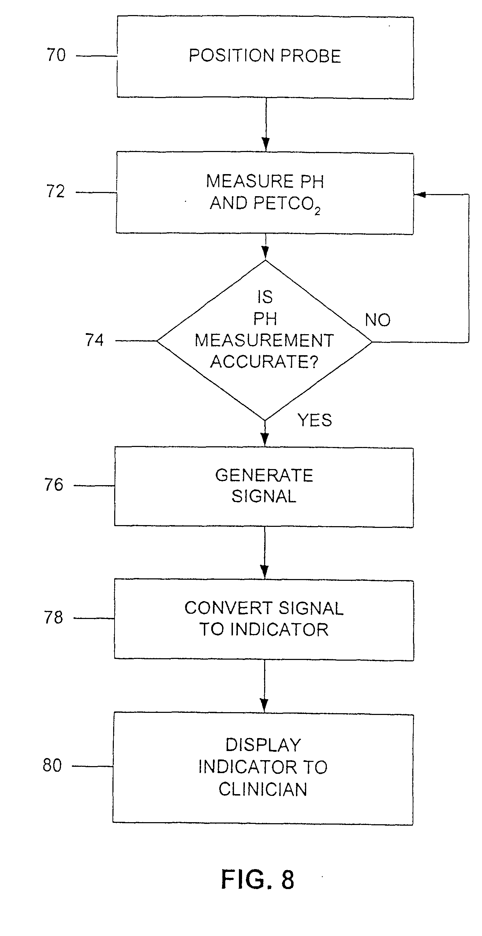

FIG. 8 is a flow chart illustrating another preferred embodiment of the method of the invention including the additional step of acquiring a $P_{etCO2}$ measurement. Generally, mucosa tissue pH correlates to arterial perfusion, while $P_{etCO2}$ correlates to pulmonary perfusion. In order to compare the arterial and pulmonary levels of perfusion failure, the mucosa tissue pH measurement is compared to the $P_{etCO2}$ measurement. Thus, the comparison between the pH and $P_{etCO2}$ measurements helps to provide a more accurate assessment of systemic perfusion failure.

Referring to FIGS. 1 and 8, the holder 14 coupled to the probe 12 is positioned 70 on the patient 10. Preferably, probe 12 includes both a pH sensor 30 and a $P_{etCO2}$ sensor (not shown). Preferably, the holder 14 is attached to the patient's cheek. In other embodiments (not shown), the holder 14 may be attached to the patient's lip or under the patient's tongue. The pH sensor 30 measures 72 the pH of the mucosa tissue, and the $P_{etCO2}$ sensor measures 72 the $P_{etCO2}$ level of the air expired by the patient. The $P_{etCO2}$ measurement is compared to the pH measurement and used as a reference to determine the accuracy of the pH measurement. If the mucosa tissue pH measurement is significantly different from the $P_{etCO2}$ measurement, which may occur in very low perfusion states, the pH measurement can be taken again 72 or the $P_{etCO2}$ measurement can be relied upon to indicate the level of perfusion failure. Once the pH measurement is accurate, the sensors generate 76 an electrical signal in response to the detected pH and $P_{etCO2}$. The signal is converted 78 into an indicator of the level of perfusion. The indicator of the level of perfusion is displayed 80 to a clinician on display unit 16.

For completeness, various aspects of the invention are set out in the following numbered clauses:

1. A device for assessing perfusion failure of a patient (10), the device comprising:

a probe (12) for contacting mucosa tissue in the upper respiratory/digestive tract of the patient (10); and
a sensor (30) coupled to the probe (12) for directly detecting a pH measurement of the mucosa tissue and for generating an electrical signal in response to the detected pH measurement.

2. The device of clause 1 wherein the sensor (30) is coupled to a steady-state device.

3. The device of clause 2 wherein the steady-state device is an ion-selective, field-effect transistor.

4. The device of clause 1 wherein the sensor (30) is an electrochemical sensor.

5. The device of clause 1 wherein the sensor (30) is not encapsulated within a permeable membrane.

6. The device of clause 1 and further comprising a second sensor for acquiring an end-tidal carbon-dioxide partial-pressure measurement.

7. The device of clause 6 wherein the pH measurement is compared to the end-tidal carbon-dioxide partial-pressure measurement.

8. The device of clause 7 wherein the end-tidal carbon-dioxide partial-pressure measurement is used as a reference to increase the accuracy of the pH measurement.

9. The device of clause 6 wherein the pH measurement correlates to arterial perfusion and the end-tidal carbon-dioxide partial-pressure measurement correlates to pulmonary perfusion.

10. The device of clause 6 wherein the sensor (30) for acquiring the pH measurement and the second sensor for acquiring the end-tidal carbon-dioxide partial-pressure measurement are coupled to the same probe (12).

11. The device of clause 1 and further comprising a reference electrode (54) connected to the sensor (30) and in contact with the mucosa tissue.

12. The device of clause 11 wherein the reference electrode (34) is a silver/silver-chloride electrode.

13. The device of clause 1 and further comprising a holder (14) for the probe (12) to secure the probe (12) to the patient (10).

14. The device of clause 13 wherein the holder (14) has an inner holder portion (90) and an outer holder portion (92), and wherein the inner holder portion (90) is coupled to the probe (12)and is placed inside the patient's mouth in contact with the oral mucosa and the outer holder portion (92) is placed outside the patient's mouth.

15. The device of clause 14 wherein the inner holder portion (90) is placed inside the patient's mouth in contact with the oral mucosa of the patient's cheek.

16. The device of clause 14 wherein the inner holder portion (90) is placed inside the patient's mouth in contact with the oral mucosa of the patient's lip.

17. The device of clause 13 wherein the holder (14) is placed under the patient's tongue.

18. The device of clause 1 wherein the sensor (30) detects the chemistry of the patient's saliva and generates an electrical signal in response to the detected saliva chemistry.

19. A device for assessing perfusion failure of a patient, the device comprising:

> a probe (12) for contacting mucosa tissue in the upper respiratory/digestive tract of the patient (10);
> a sensor (30) coupled to the probe (12) for directly acquiring a pH measurement of the mucosa tissue;
> a reference electrode (34) connected to the sensor (30) and in contact with the mucosa tissue so that an electrical potential correlating to a level of tissue perfusion is created between the sensor (30) and the reference electrode (34).

20. The device of clause 19 wherein the sensor (30) is coupled to a steady-state device.

21. The device of clause 20 wherein the steady-state device is an ion-selective, field-effect transistor.

22. The device of clause 19 wherein the sensor (30) is an electrochemical sensor.

23. The device of clause 19 wherein the sensor (30) is not encapsulated with a permeable membrane.

24. The device of clause 19 wherein the reference electrode (34) is a silver/silver-chloride electrode.

25. The device of clause 19 and further comprising a second sensor for acquiring an end-tidal carbon-dioxide partial-pressure measurement.

26. The device of clause 25 wherein the pH measurement is compared to the end-tidal carbon-dioxide partial-pressure measurement.

27. The device of clause 26 wherein the end-tidal carbon-dioxide partial-pressure measurement is used as a reference to increase the accuracy of the pH measurement.

28. The device of clause 25 wherein the pH measurement correlates to arterial perfusion and the end-tidal carbon-dioxide partial-pressure measurement correlates to pulmonary perfusion.

29. The device of clause 25 wherein the sensor (30) for acquiring the pH measurement and the second sensor for acquiring the end-tidal carbon-dioxide partial-pressure measurement are coupled to the same probe (12).

30. The device of clause 19 and further comprising a holder (14) for the probe (12) to secure the probe (12) to the patient (10).

31. The device of clause 30 wherein the holder (14) has an inner holder portion (90) and an outer holder portion (92), and wherein the inner holder portion (90) is coupled to the probe (12) and is placed inside the patient's mouth in contact with the oral mucosa and the outer holder portion (92) is placed outside the patient's mouth.

32. The device of clause 31 wherein the inner holder portion (90) is placed inside the patient's mouth in contact with the oral mucosa of the patient's cheek.

33. The device of clause 31 wherein the inner holder portion (90) is placed inside the patient's mouth in contact with the oral mucosa of the patient's lip.

34. The device of clause 31 wherein the holder (14) is placed under the patient's tongue.

35. The device of clause 19 wherein the sensor (30) detects the chemistry of the patient's saliva and generates an electrical signal in response to the detected saliva chemistry.

36. A method of assessing tissue perfusion in a patient, the method comprising the acts of:

> providing a probe (12) capable of measuring pH;
> placing the probe (12) in contact with mucosa tissue in the upper digestive/respiratory tract of the patient (10);
> obtaining a pH measurement of the mucosa tissue; and
> converting the pH measurement into an indicator to a clinician representing a level of tissue perfusion.

37. The method of clause 36 wherein the probe (12) includes a sensor (30) for measuring pH.

38. The method of clause 36 wherein the sensor (30) is coupled to a steady-state device.

39. The method of clause 38 wherein the steady-state device is an ion-selective, field-effect transistor.

40. The method of clause 36 wherein the sensor (30) is an electrochemical sensor.

41. The method of clause 36 and further comprising the acts of

providing a probe (12) capable of measuring end-tidal carbon-dioxide partial-pressure;

placing the probe (12) in the upper digestive/respiratory tract; and

obtaining an end-tidal carbon-dioxide partial-pressure measurement.

42. The method of clause 41 and further comprising the act of comparing the pH measurement to the end-tidal carbon-dioxide partial-pressure measurement.

43. The method of clause 42 wherein the end-tidal carbon-dioxide partial-pressure measurement is compared to the pH measurement as a reference to increase the accuracy of the pH measurement.

44. The method of clause 41 wherein the pH measurement correlates to arterial perfusion and the end-tidal carbon-dioxide partial-pressure measurement correlates to pulmonary perfusion.

45. The method of clause 41 wherein the probe (12) capable of measuring pH and the probe (12) capable of measuring end-tidal carbon-dioxide partial-pressure are the same probe (12).

46. The method of clause 36 wherein the act of obtaining a pH measurement of the mucosa tissue further comprises the act of generating an electrical signal indicative of the pH of the mucosa tissue.

47. The method of clause 36 and further comprising the acts of introducing a holder (14) coupled to the probe (12) into the patient's upper digestive/respiratory tract and securing the holder (14) to the patient (10).

48. The method of clause 47 wherein the holder (14) has an inner holder portion (90) coupled to the probe (12) and an outer holder portion (92).

49. The method of clause 48 and further comprising the acts of placing the inner holder portion (90) inside the patient's mouth and placing the outer holder portion outside the patient's mouth.

50. The method of clause 49 wherein the act of placing the inner holder portion (90) inside the patient's mouth further comprises the act of placing the inner holder portion (90) in contact with the oral mucosa of the patient's cheek.

51. The method of clause 49 wherein the act of placing the inner holder portion (90) inside the patient's mouth further comprises the act of placing the inner holder portion (90) in contact with the oral mucosa of the patient's lip.

52. The method of clause 49 wherein the act of placing the inner holder portion (90) inside the patient's mouth further comprises the act of placing the inner holder portion (90) in contact with the oral mucosa under the patient's tongue.

53. The method of clause 36 and further comprising

providing a probe (12) capable of measuring the patient's saliva chemistry;

placing the probe (12) in contact with the patient's saliva;

obtaining a saliva-chemistry measurement; and

converting the saliva-chemistry measurement into an indicator to a clinician.

54. The method of clause 53 wherein the probe (12) capable of measuring pH and the probe (12) capable of measuring the patient's saliva chemistry are the same probe (12).

55. A device for assessing the blood chemistry of a patient, the device comprising:

a probe (12) for contacting mucosa tissue in the upper respiratory/digestive tract of the patient (10);

a sensor (30) coupled to the probe (12) for detecting blood-chemistry data for the mucosa tissue and for generating a signal in response to the detected blood-chemistry data.

56. The device of clause 55 wherein the blood-chemistry data is the data gathered for an arterial blood gas analysis.

57. The device of clause 55 wherein the sensor (30) is coupled to a steady-state device.

58. The device of clause 57 wherein the steady-state device is an ion-selective, field-effect transistor.

59. The device of clause 55 wherein the sensor (30) is an electrochemical sensor.

60. The device of clause 55 and further comprising a second sensor for acquiring an end-tidal carbon-dioxide partial-pressure measurement.

61. The device of clause 60 wherein the pH measurement is compared to the end-tidal carbon-dioxide partial-pressure measurement.

62. The device of clause 61 wherein the end-tidal carbon-dioxide partial-pressure measurement is compared to the pH measurement as a reference to increase the accuracy of the pH measurement.

63. The device of clause 60 wherein the pH measurement correlates to arterial perfusion and the end-tidal carbon-dioxide partial-pressure measurement correlates to pulmonary perfusion.

64. The device of clause 60 wherein the sensor (30) for acquiring the blood-chemistry data and the second sensor for acquiring the end-tidal carbon-dioxide partial-pressure measurement are included in the same probe (12).

65. The device of clause 55 wherein the sensor (30) detects the chemistry of the patient's saliva and generates an electrical signal in response to the detected saliva chemistry.

66. A device for assessing the blood chemistry of a patient (10), the device comprising:

   a probe (12) for contacting mucosa tissue in the upper respiratory/digestive tract of the patient (10);
   a first sensor (80) coupled to the probe for detecting the data gathered for an arterial blood gas analysis and for generating a signal in response to the detected arterial blood gas data; and
   a second sensor coupled to the probe (12) for acquiring an end-tidal carbon-dioxide partial-pressure measurement.

67. The device of clause 66 wherein the first sensor (30) is coupled to a steady-state device.

68. The device of clause 67 wherein the steady-state device is an ion-selective, field-effect transistor.

69. The device of clause 66 wherein the first sensor (30) is an electrochemical sensor.

70. The device of clause 66 wherein the data gathered for an arterial blood gas analysis includes a pH measurement.

71. The device of clause 70 wherein the pH measurement is compared to the end-tidal carbon-dioxide partial-pressure measurement.

72. The device of clause 71 wherein the end-tidal carbon-dioxide partial-pressure measurement is compared to the pH measurement as a reference to increase the accuracy of the pH measurement.

73. The device of clause 70 wherein the pH measurement correlates to arterial perfusion and the end-tidal carbon-dioxide partial-pressure measurement correlates to pulmonary perfusion.

74. The device of clause 66 wherein the first sensor (30) detects the chemistry of the patient's saliva and generates an electrical signal in response to the detected saliva chemistry.

75. A method of assessing tissue perfusion in a patient, the method comprising the acts of:

   providing a probe (12) capable of measuring pH and end-tidal carbon-dioxide partial-pressure;
   placing the probe (12) in the patient's upper digestive/respiratory tract; obtaining a pH measurement and an end-tidal carbon-dioxide partial-pressure measurement;
   generating an electrical signal indicative of the pH measurement and the end-tidal carbon-dioxide partial-pressure measurement; and
   converting the electrical signal into an indicator to a clinician representing a level of tissue perfusion.

76. The method of clause 75 wherein the probe (12) includes a sensor (30) for measuring pH.

77. The method of clause 76 wherein the sensor (30) is coupled to a steady-state device.

78. The method of clause 77 wherein the steady state device is an ion-selective, field-effect transistor.

79. The method of clause 76 wherein the sensor (30) is an electrochemical sensor.

80. The method of clause 75 and further comprising the act of comparing the pH measurement to the end-tidal carbon-dioxide partial-pressure measurement.

81. The method of clause 80 wherein the end-tidal carbon-dioxide partial-pressure measurement is compared to the pH measurement as a reference to increase the accuracy of the pH measurement.

82. The method of clause 75 wherein the pH measurement correlates to arterial perfusion and the end-tidal carbon-dioxide partial-pressure measurement correlates to pulmonary perfusion.

83. The method of clause 75 wherein the probe (12) is capable of measuring the patient's saliva chemistry.

84. The method of clause 83 and further comprising placing the probe (12) in contact with the patient's saliva;

obtaining a saliva-chemistry measurement;

generating an electrical signal indicative of the saliva-chemistry measurement; and

converting the saliva-chemistry measurement into an indicator to a clinician.

85. A patient monitor comprising:

a processing and display unit (16); and a device (11) for assessing perfusion failure of the patient, the device including a probe (12) for contacting mucosa tissue in the upper respiratory/digestive tract of the patient and a sensor (30) coupled to the probe (12) for directly detecting a pH measurement of the mucosa tissue and for generating an electrical signal in response to the detected pH measurement.

86. The device of clause 85 wherein the sensor (30) is coupled to a steady-state device.

87. The device of clause 86 wherein the steady-state device is an ion-selective, field-effect transistor.

88. The device of clause 85 wherein the sensor (30) is an electrochemical sensor.

89. The device of clause 85 wherein the sensor (30) is not encapsulated within a permeable membrane.

90. The device of clause 85 and further comprising a second sensor for acquiring an end-tidal carbon-dioxide partial-pressure measurement.

91. The device of clause 90 wherein the pH measurement is compared to the end-tidal carbon-dioxide partial-pressure measurement.

92. The device of clause 91 wherein the end-tidal carbon-dioxide partial-pressure measurement is used as a reference to increase the accuracy of the pH measurement.

93. The device of clause 90 wherein the pH measurement correlates to arterial perfusion and the end-tidal carbon-dioxide partial-pressure measurement correlates to pulmonary perfusion.

94. The device of clause 90 wherein the sensor (30) for acquiring the pH measurement and the second sensor for acquiring the end-tidal carbon-dioxide partial-pressure measurement are coupled to the same probe.

95. The device of clause 85 and further comprising a reference electrode (34) connected to the sensor and in contact with the patient's mucosa tissue.

96. The device of clause 95 wherein the reference electrode (34) is a silver/silver-chloride electrode.

97. The device of clause 85 and further comprising a holder (14) for the probe (12) to secure the probe (12) to the patient.

98. The device of clause 97 wherein the holder (14) has an inner holder portion (90) and an outer holder portion (92), and wherein the inner holder portion (90) is coupled to the probe (12) and is placed inside the patient's mouth in contact with the oral mucosa and the outer holder portion (92) is placed outside the patient's mouth.

99. The device of clause 98 wherein the inner holder portion (90) is placed inside the patient's mouth in contact with the oral mucosa of the patient's cheek.

100. The device of clause 98 wherein the inner holder portion (90) is placed inside the patient's mouth in contact with the oral mucosa of the patient's lip.

101. The device of clause 97 wherein the holder (14) is placed under the patient's tongue.

102. The device of clause 85 wherein the sensor (30) detects the chemistry of the patient's saliva and generates an electrical signal in response to the detected saliva chemistry.

**Claims**

1. A device for assessing perfusion failure of a patient (10), the device comprising:

a probe (12) for contacting mucosa tissue in the upper respiratory/digestive tract of the patient (10); and a sensor (30) coupled to the probe (12) for directly detecting a pH measurement of the mucosa tissue and for generating an electrical signal in response to the detected pH measurement.

**2.** A device for assessing perfusion failure of a patient, the device comprising:

a probe (12) for contacting mucosa tissue in the upper respiratory/digestive tract of the patient (10);
a sensor (30) coupled to the probe (12) for directly acquiring a pH measurement of the mucosa tissue;
a reference electrode (34) connected to the sensor (30) and in contact with the mucosa tissue so that an electrical potential correlating to a level of tissue perfusion is created between the sensor (30) and the reference electrode (34).

**3.** A device for assessing the blood chemistry of a patient, the device comprising:

a probe (12) for contacting mucosa tissue in the upper respiratory/digestive tract of the patient (10); and
a sensor (30) coupled to the probe (12) for detecting blood-chemistry data for the mucosa tissue and for generating a signal in response to the detected blood-chemistry data.

**4.** A device for assessing the blood chemistry of a patient (10), the device comprising:

a probe (12) for contacting mucosa tissue in the upper respiratory/digestive tract of the patient (10);
a first sensor (80) coupled to the probe for detecting the data gathered for an arterial blood gas analysis and for generating a signal in response to the detected arterial blood gas data; and
a second sensor coupled to the probe (12) for acquiring an end-tidal carbon-dioxide partial-pressure measurement.

**5.** The device of any one of claims 1 to 4 wherein the sensor (30) is coupled to a steady-state device comprising an ion-selective, field-effect transistor.

**6.** The device of any one of claims 1 to 4 wherein the sensor (30) is an electrochemical sensor.

**7.** The device of any one of claims 1 to 4 wherein the sensor (30) is not encapsulated within a permeable membrane.

**8.** A patient monitor comprising:

a processing and display unit (16); and

a device (11) for assessing perfusion failure of the patient, said device being in accordance with any one of claim 1 to 7.

**9.** A method of assessing tissue perfusion in a patient, the method comprising the acts of:

providing a probe (12) capable of measuring pH;
placing the probe (12) in contact with mucosa tissue in the upper digestive/respiratory tract of the patient (10);
obtaining a pH measurement of the mucosa tissue; and
converting the pH measurement into an indicator to a clinician representing a level of tissue perfusion.

**10.** A method of assessing tissue perfusion in a patient, the method comprising the acts of:

providing a probe (12) capable of measuring pH and end-tidal carbon-dioxide partial-pressure;
placing the probe (12) in the patient's upper digestive/respiratory tract; obtaining a pH measurement and an end-tidal carbon-dioxide partial-pressure measurement;
generating an electrical signal indicative of the pH measurement and the end-tidal carbon-dioxide partial-pressure measurement; and
converting the electrical signal into an indicator to a clinician representing a level of tissue perfusion.

Fig. 1

DISPLAY UNIT

Fig. 5

Fig. 6

Fig. 7

FIG. 2

30

32

VOLTMETER

44

| 34 | 42 | 38 | 36 |
|---|---|---|---|
| REFERENCE ELECTRODE | SAMPLE (UNKNOWN PH) | BUFFER SOLUTION (CONSTANT PH) | AG/AGCI REFERENCE WIRE |

PH-SENSITIVE GLASS MEMBRANE

40

## FIG. 3

30

62

66

64        64

54

DRAIN        56        SOURCE

$I_{ds}$

58        52        60

$V_{gs}$

$V_{ds}$

Fig. 1

**FIG. 8**

### European Patent Office

## PARTIAL EUROPEAN SEARCH REPORT

**Application Number**

which under Rule 45 of the European Patent Convention EP 01 31 0531
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | OESCH U ET AL: "Solvent polymeric membrane pH catheter electrode for intraluminal measurements in the upper gastrointestinal tract" MEDICAL & BIOLOGICAL ENGINEERING & COMPUTING, JULY 1987, UK, vol. 25, no. 4, pages 414-419, XP002195504 ISSN: 0140-0118 | 1-3,6-8 | A61B5/00 |
| A | * section 1 "Introduction" * | 4,5 | |
| X | WO 99 16346 A (INST OF CRITICAL CARE MEDICINE) 8 April 1999 (1999-04-08) | 1,3-8 | |
| A | * page 16, line 13 - line 26 * | 2 | |
| | -/-- | | |

**TECHNICAL FIELDS SEARCHED (Int.Cl.7)**

A61B

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

9,10

Reason for the limitation of the search:

Article 52 (4) EPC - Method for treatment of the human
or animal body by surgery
Article 52 (4) EPC - Diagnostic method practised on
the human or animal body

| Place of search | Date of completion of the search | Examiner |
|---|---|---|
| THE HAGUE | 9 April 2002 | Knüpling, M |

EPO FORM 1503 03 82 (P04C07)

EP 1 219 234 A1

European Patent
Office

Application Number

EP 01 31 0531

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

18

**European Patent Office**

**PARTIAL EUROPEAN SEARCH REPORT**

Application Number

EP 01 31 0531

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | OGINO H ET AL: "Reflectance pulse oximeter measuring central SaO2 from mouth" ENGINEERING IN MEDICINE AND BIOLOGY SOCIETY, 1994. ENGINEERING ADVANCES: NEW OPPORTUNITIES FOR BIOMEDICAL ENGINEERS., PROCEEDINGS OF THE 16TH ANNUAL INTERNATIONAL CONFERENCE OF THE IEEE BALTIMORE, MD, USA 3-6 NOV. 1994, NEW YORK, NY, USA,IEEE, US, 3 November 1994 (1994-11-03), pages 914-915, XP010145703 ISBN: 0-7803-2050-6 | 3,6-8 | |
| A | * section "Introduction" * | 1,2,4 | **TECHNICAL FIELDS SEARCHED** (Int.Cl.7) |
| X | US 4 629 424 A (LAUKS IMANTS R ET AL) 16 December 1986 (1986-12-16) * column 1, line 19 - line 44 * | 1-3,5-8 | |
| A | * column 4, line 5 - line 12 * | 4 | |
| X | EP 0 882 434 A (TAKEUCHI HIDEYUKI; KIRINO SHIGERU (JP)) 9 December 1998 (1998-12-09) * column 3, line 15 - line 21 * | 1,3,5-8 | |
| A | * column 4, line 25 - line 28 * | 2,4 | |

**European Patent Office**

**LACK OF UNITY OF INVENTION
SHEET B**

Application Number

EP 01 31 0531

The Search Division considers that the present European patent application does not comply with the requirements of unity of invention and relates to several inventions or groups of inventions, namely:

1. Claims: 1,2

   Sensor for detecting pH of mucosa tissue

2. Claim : 3

   Sensor for detecting blood-chemistry of mucosa

3. Claim : 4

   Probe having two sensors for blood gas analysis and carbon-dioxide partial pressure measurement

**ANNEX TO THE EUROPEAN SEARCH REPORT
ON EUROPEAN PATENT APPLICATION NO.**                    EP 01 31 0531

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2002

| Patent document cited in search report | | Publication date | Patent family member(s) | | Publication date |
|---|---|---|---|---|---|
| WO 9916346 | A | 08-04-1999 | US | 6055447 A | 25-04-2000 |
| | | | AU | 740775 B2 | 15-11-2001 |
| | | | AU | 9509598 A | 23-04-1999 |
| | | | CA | 2304534 A1 | 08-04-1999 |
| | | | EP | 1018933 A1 | 19-07-2000 |
| | | | WO | 9916346 A1 | 08-04-1999 |
| | | | US | 6216024 B1 | 10-04-2001 |
| | | | US | 6258046 B1 | 10-07-2001 |
| | | | US | 2001025151 A1 | 27-09-2001 |
| US 4629424 | A | 16-12-1986 | NONE | | |
| EP 0882434 | A | 09-12-1998 | JP | 10328212 A | 15-12-1998 |
| | | | EP | 0882434 A2 | 09-12-1998 |
| | | | US | 5947726 A | 07-09-1999 |